## Europäisches Patentamt
## European Patent Office
## Office européen des brevets

(19)

(11) Publication number: **0 043 461 B1**

(12) **EUROPEAN PATENT SPECIFICATION**

(45) Date of publication of patent specification: **22.02.84**

(51) Int. Cl.³: **A 61 N 1/04, C 25 D 5/50**

(21) Application number: **81104509.5**

(22) Date of filing: **11.06.81**

(54) **Process for manufacturing electrodes for cardiac stimulators.**

(30) Priority: **19.06.80 IT 6796280**

(43) Date of publication of application:
**13.01.82 Bulletin 82/2**

(45) Publication of the grant of the patent:
**22.02.84 Bulletin 84/8**

(84) Designated Contracting States:
**DE FR GB NL**

(56) References cited:
**US - A - 2 847 372**
**US - A - 3 749 101**
**US - A - 4 011 861**
**US - A - 4 156 429**

**PROCEEDINGS OF THE IEEE, vol. 56, no. 6, June 1968 D.A. ROBINSON "The electrical properties of metal microelectrodes", pages 1065-1071**
**PRODUCT ENGINEERING, vol. 49, no. 5, May 1978 New York, US "Pacemaker challenges design ingenuity", page 14**

(73) Proprietor: **SORIN BIOMEDICA S.p.A.**
**Strada per Crescentino**
**I-13040 Saluggia (Vercelli) (IT)**

(72) Inventor: **Freud, Gerrit Ernst**
**Keizersgracht 694**
**NL-1017 EV Amsterdam (NL)**
Inventor: **Chinaglia, Benito**
**Via Moncalvo 33**
**I-10131 Torino (IT)**
Inventor: **Cerise, Osvaldo**
**Via Planaville 11**
**I-11010 Doues (Aosta) (IT)**

(74) Representative: **Jacobacci, Filippo et al,**
**c/o JACOBACCI-CASETTA & PERANI S.p.A. Via Alfieri 17**
**I-10121 Torino (IT)**

Courier Press, Leamington Spa, England.

## Process for manufacturing electrodes for cardiac stimulators

This invention refers to a process for manufacturing electrodes for cardiac stimulators ("pacemaker") comprising a metallic tip with a porous surface of contact with the cardiac wall.

As compared with a smooth surface tip, a porous surface tip presents several advantages, such as:

— a good fastening to the cardiac wall owing to a better growth of the cardiac tissue on the porous substrate

— low polarization losses owing to increased contact surface;

— for a given pulse from the pacemaker, a greater current directly transmitted to the cells of the cardiac muscle and therefore a wider safety margin;

— a lower voltage threshold, which permits the use of impulses at a lower energy level without decreasing the safety margin;

— increased sensitivity of the electrode to the endocardic signals thanks to a lower attenuation of the latter at their passage from the cells to the tip.

The known tips with porous contact layer are constituted by stainless steel. Other medical electrodes having tips with porous platinum contacts formed by electrolysis are disclosed in US—A—3 749 101 and in the proceedings of the IEEE, vol. 56, no. 6, June 1968 pages 1065—1071.

An object of the present invention is to provide a process for manufacturing electrodes with improved contact tip, able to further enhance the above recalled advantages.

In the electrode made by the process according to the invention the contact tip comprises a body of platinum/iridium alloy coated with platinum sponge. The diameter of the pores of the platinum sponge is advantageously of the order of 40 microns. The thickness of the coating is preferably of the order of 0.1 mm; greater thickness values don't improve the result.

Still preferably, the weight ratio platinum/iridium in the alloy which forms the body of the tip is substantially 90:10.

The process according to the invention is basically characterized in that the coating of platinum sponge on the body of platinum/iridium is formed by porous layer electrolytic deposition (as distinct from compact layer deposition), whereupon the obtained electrolytic layer is consolidated by sintering.

The electrolyte preferably consists of chloroplatinic acid $H_2PtCl_6 \cdot H_2O$ dissolved in deionized water in a proportion of about 3g per 100 cc water. The applied voltage on the electrolyzer can vary from 2 to 10 V. In order to obtain a platinum deposit of desired porosity, the current density should be maintained in the range of 1—2 Amp/sq.cm, preferably 1.5 Amp/sq.cm.

The necessary thickness of the coating is obtained in a time from 1 to 5 minutes. After rinsing it is possible to proceed with sintering, preferably at about 1300°C for about 20 minutes. In the sintering furnace, or during the subsequent cooling of the tip, no particular protective atmosphere is necessary.

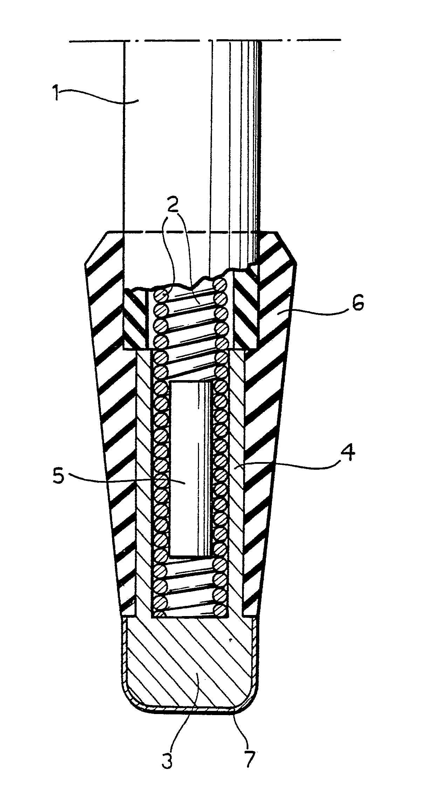

An electrode made by a process according to the invention is illustrated in axial cross-sectional view in the enclosed drawing.

Reference 1 denotes a sheath (of silicone rubber) of an endocardiac probe, containing an electric conductor 2 in form of a helical spiral, which can be monofilament but preferably is composed of three filaments for reliability reasons, according to the known art. Reference 3 denotes the body of the tip of the electrode, from which a tubular shank 4 extends towards the sheath 1. The body 3 and the shank 4 are of platinum/iridium 90/10 weight ratio. The spiral 2 extends beyond the sheath 1 and penetrates into the shank 4, which is crimped on a length of the spiral containing a small metallic cylinder 5 acting as "anvil" or "mandrel" against the crushing of the spiral during crimping. The shank 4 and the adjacent end portion of the sheath 1 are covered by a silicone rubber sleeve 6.

The body 3 forming a sort of mushroom on the shank 4, is coated with a porous platinum layer, denoted by 7, having pores of a diameter of about 40 mircrons. For obtaining such layer, the body 3 was dipped into an electrolyte as hereinbefore described. The electrolysis was carried out in 5 minutes, with a voltage of 2.5 Volt and a current density of 1.5 Amp/sq.cm. The obtained coating was then sintered at 1300°C for 20 minutes.

## Claims

1. Process for the manufacture of an electrode for a cardiac stimulator comprising a metallic tip (3) constituted by a body of a platinum/iridium alloy and a coating of platinum sponge forming on the tip a porous surface for contact with the cardiac wall, wherein the said coating is formed on said body by electrolytic deposition, characterized by the step of consolidating the electrolytically deposited porous layer by sintering.

2. The process of claim 1, characterized in that sintering is effected at about 1300°C for about 20 minutes.

3. The process of claims 3, characterized in that the weight ratio platinum/iridium in the alloy is about 90:10.

4. The process according to any of claims 1—4, characterized in that the electrolyte for electrolyte deposition is formed from chloroplatinic acid hexahydrate and deionized water in a proportion of 3g acid *per* 100 ml water, and in that the electrolysis is effected at a current density of 1—2 Ampères/sq.cm. during a time of 1—5 minutes.

## Revendications

1. Procédé pour la fabrication d'une électrode pour un stimulateur cardiaque, comprenant une extrémité métallique (3) constituée d'un corps en alliage platine/iridium et un revêtement d'éponge de platine formant sur l'extrémité une surface poreuse pour le contact avec le paroi cardiaque, où ledit revêtement est formé sur ledit corps par déposition électrolytique, caractérisé par l'étape de consolidation par frittage de la couche poreuse déposée electrolytiquement.

2. Le procédé de la revendication 1, caractérisé en ce que le frittage est effectué à environ 1300°C pendant environ 20 min.

3. Le procédé de la revendication 3, caractérisé en ce que le rapport pondéral platine/iridium dans l'alliage est d'environ 90:10.

4. Le procédé selon l'une quelconque des revendications 1 à 4, caractérisé en ce que l'electrolyte pour la déposition électrolytique est formé d'acide chloroplatinique hexahydraté et d'eau désionisée dans une proportion de 3 g d'acide par 100 ml d'eau et en ce que l'électrolyse est effectuée à une densité de courant de 1—2 A/cm² pendant une durée de 1—5 min.

## Patentansprüche

1. Verfahren zur Herstellung einer Elektrode für einen Herzstimulator mit einer metallischen Spitze (3), die von einem aus einer Platin-Iridium-Legierung bestehenden Körper mit einer Beschichtung (7) aus Platinschwamm gebildet ist, die an der Spitze (3) eine poröse Fläche für den Kontakt mit der Herzwand bildet, wobei die genannte Bechichtung durch elektrolytische Abscheidung auf dem Körper aufgebracht ist, gekennzeichnet durch einen Verfahrensschritt der Verfestigung der elektrolytisch abgeschiedenen porösen Beschichtung durch Sintern.

2. Verfahren nach Anspruch 1, dadurch gekennzeichnet, daß das Sintern bei einer Temperatur von etwa 1300°C während einer Zeitdauer von etwa 20 Minuten durchgeführt wird.

3. Verfahren nach Anspruch 1 oder 2, dadurch gekennzeichnet, daß das Platin-Iridium-Gewichtsverhältnis in der genannten Legierung etwa 90:10 beträgt.

4. Verfahren nach einem der Ansprüche 1 bis 3, dadurch gekennzeichnet, daß der Elektrolyt für die elektrolytische Abscheidung, aus Chlorplatinsäurehexahydrat und deionisiertem Wasser ($H_2PtCl_6.H_2O$) in einem Verhältnis von 3 g Säure pro 100 ml Wasser besteht und daß die Elektrolyse mit einer Stromdichte von 1 bis 2 Ampere/cm² während einer Zeit von 1 bis 5 Minuten durchgeführt wird.

1